# EUROPEAN PATENT APPLICATION

(11) **EP 1 870 128 A1**
(43) Date of publication of application: **26.12.2007**
(21) Application number: 06425417.0
(22) Date of filing: 19.06.2006
(51) Int. Cl.: A61N 1/34, A61N 1/36

(54) **Analgesic neuromodulating device, with a modulating effect depending on the user's activity and position**

(71) Applicant: Lifestim S.r.l., 35030 Rubano (PD) (IT)
(72) Inventor: Snichelotto, Eugenio, c/o Lifestim S.r.l., 35030 Rubano (PD) (IT)
(74) Representative: Vinci, Marcello

(57) **Abstract**

The invention is a new implantable analgesic neuromodulating device comprising a casing (1) which houses at least one battery (6), one control and command circuit (2), and wherein said control and command circuit (2) is connected to one or more terminals (5), outside the casing (1), suitable for performing the neurostimulation, comprising a movement detection device or accelerometer (4) connected to said control and command circuit (2), and wherein said control and command circuit (2) analyses the signals generated by said movement detection device or accelerometer (4) and activates, interrupts or modifies administration of the neurostimulation.

## Description

The present patent relates to implantable devices and in particular concerns analgesic neurostimulation systems for the treatment of chronic pain.

Pain is a symptom that is difficult to quantify and the sensation of pain is transmitted to the brain via specific nerve fibres.

It is known that analgesic stimulation neutralises or in any case alleviates the sensation of pain in cases of chronic pain.

The neuromodulation systems for the treatment of pain are electronic devices which stimulate one or more nerve fibres.

With said stimulation the user experiences a sensation of "touching" instead of a sensation of pain.

In cases of chronic pain, implantable neuromodulation devices, similar to pacemakers, which stimulate one or more nerve fibres, are applied.

The analgesic neurostimulation performed by said implantable devices is administered via electrodes applied at the level of the backbone or spinal column.

The stimulation energy necessary to obtain an effective analgesic action is, however, normally much greater than that required for a pacemaker, with the result that neuromodulating devices, with respect to pacemakers, have various drawbacks.

The analgesic neurostimulators require considerable energy and consequently their batteries have a short life, much less than the normal life of the batteries used for pacemakers.

It is possible to use batteries with higher capacity but this increases the overall dimensions and weight of the neurostimulation system.

The analgesic neurostimulators do not operate continuously but are used only at moments or periods in which the pain is manifest or unbearable.

Situations frequently occur in which analgesic stimulation can be usefully administered only in certain conditions of activity or non-activity of the user.

In most cases, in fact, the pain appears during the night.

The analgesic neurostimulators are switched on and off manually, by placing a magnet close to the switch implanted with the neurostimulation device. Some devices are activated by means of remote control.

Manual activation of the neuromodulator during the night, in particular by means of the switch-on magnet, can be a difficult operation or an operation that is difficult to remember, especially for the elderly.

The neurostimulation is performed by appropriately stimulating nerve bundles of the central nervous system, or by stimulating specific nerve bundles of the spinal marrow.

The spinal marrow is encased and protected by the three meninges, the outer one of which, the dura mater, is thicker.

The spinal marrow is subject to the force of gravity and tends to bend inside the dura mater, modifying its position with respect to the dura mater according to the position assumed by the person.

For example, if the user is standing or sitting, the nerve bundles of the marrow are arranged in a normal position inside the dura mater, if the user is lying face up, the nerve bundles of the marrow move by gravity towards the rear wall of the dura mater, if the user is lying face down the nerve bundles of the marrow move by gravity towards the front wall of the dura mater, and if the user is lying on one side, the nerve bundles of the marrow move by gravity towards the lower wall of the dura mater.

The change of posture, prone or supine or on one side, modifies the distance of the spinal marrow from the neurostimulation electrode.

The greater or lesser distance of the spinal marrow and related nerve bundles from the neurostimulation electrode, even if slight, results in a different neurostimulation effect. In fact, the greater the distance of the stimulation electrode from the nerve bundle, the lesser the stimulation signal that reaches the nerve bundle, and the lesser the distance between electrode and nerve bundle, the greater the stimulation signal that reaches said nerve bundle.

It follows that, according to the position of the user, analgesic neurostimulation, by means of the known electrostimulators, can be more or less effective.

When the nerve bundle is in a position opposite to the electrode, the stimulation of the electrode is not sufficient to obtain adequate analgesic neurostimulation.

To remedy this situation it is possible to modify the parameters of the electrostimulator during programming, increasing the signal and intensity of stimulation.

On the other hand, when the nerve bundle is near the wall of the dura mater to which the electrode is applied, the stimulation of the electrode can be excessive for analgesic neurostimulation.

Increase in the signal and intensity of stimulation reduces the life of the batteries and can lead to situations of habituation of the nerve bundle to the stimulations.

To remedy all the above drawbacks, a new analgesic neuromodulating device with a modulating effect depending on the user's position and activity has been designed and produced.

One object of the new analgesic neurostimulator is to autonomously activate or de-activate neural stimulation during the various phases of activity of the user according to programmable criteria.

A further object of the new analgesic neurostimulator is to recognise the position of the user and accordingly modify the intensity of the neurostimulation.

A further object of the new analgesic neurostimulator is to autonomously modulate, again according to programmable criteria, the analgesic stimulation according to the state of activity of the user.

A further object of the new analgesic neurostimulator is to autonomously recognise the sleeping state and activate/de-activate or regulate accordingly the administration of neurostimulation.

A further object of the new analgesic neurostimulator is to provide for and use different analgesic neurostimulation patterns specific for each individual user and for each state of activity of the user such as rest, moderate activity or intense activity.

These and other direct and complementary objects have been achieved through the implementation of the new analgesic neuromodulating device with one or more accelerometers suitable for recognising the position and/or the state of activity of the user and activate/modulate administration of the appropriate neural stimulation.

A constant neurostimulation effect on the spinal marrow can be obtained by varying the intensity of the stimulation according to the posture, obtaining the effect of compensating for the variation in distance between nerve bundle and electrodes.

Accelerometers based on detection of the inertia of a mass when it undergoes an acceleration are known.

Piezoelectric accelerometers consisting of a mass connected to a piezoelectric crystal are known and very widespread.

When the mass accelerates, it applies a force to the crystal. In this way a charge, proportional to the force applied, polarises the opposite faces of the crystal. Consequently, at the output a voltage is generated that is proportional to the acceleration.

These accelerometers are usually very compact and, given their modest overall dimensions, accelerometers consisting of several piezoelectric elements, for example accelerometers that can measure accelerations along the three spatial axes, are also present on the market.

Gravitational accelerometers are known, suitable for determining the position in which they are inclined, that is, the direction of the gravitational pull.

The new analgesic neuromodulating device is provided with at least one accelerometer which has two or more axes and/or which is gravitational, which recognises the position, movements, and therefore the state of activity of the user. The signals generated by the accelerometer are analysed by the new device which compares them with parameters and tables previously stored.

The new device varies its neurostimulation pattern according to standard and/or programmable modes, according to the position, type and intensity of the user's activity and depending on the parameters and tables stored.

There is an external device by means of which, via a telemetric connection with the device implanted, the doctor can adapt the values contained in the table stored in the device according to user requirements.

The new analgesic neuromodulating device with accelerometer provides for a therapy which adopts, moment by moment, the best compromise between effectiveness obtained and energy consumed.

The characteristics of the new analgesic neuromodulating device with a modulating effect depending on the user's position and activity will be highlighted in greater detail in the following description with reference to the drawing, attached as a non-limiting example.

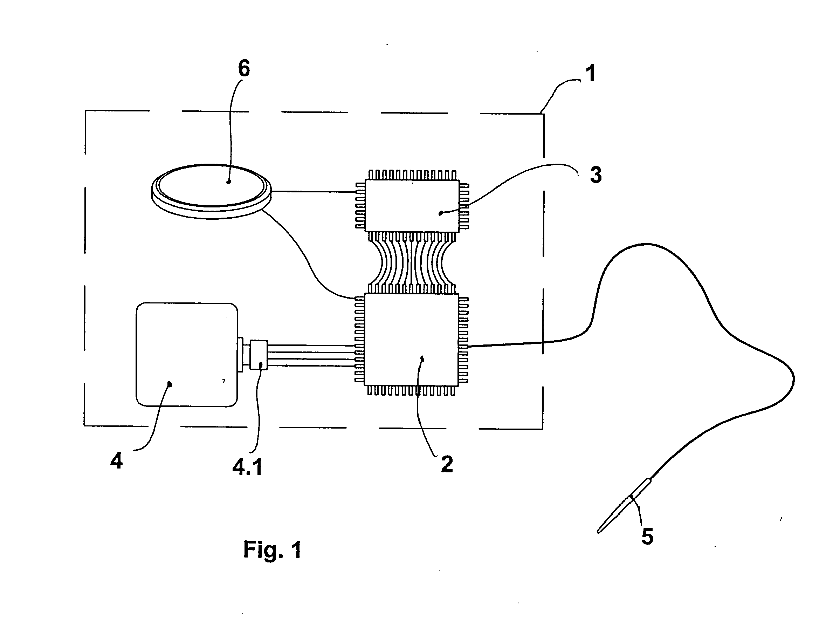

Figure 1 is a schematic view of the new analgesic neuromodulating device comprising a casing (1) which houses at least one control and command circuit (2), a battery or accumulator (6), an operating parameter memory (3) and an accelerometer (4) provided with a circuit (4.1) for interfacing with the control and command circuit (2). The control and command circuit (2) is connected to one or more neurostimulation terminals (5), outside the casing (1), suitable for performing the neurostimulation.

The accelerometer (4) is preferably of the type with at least two axes suitable for recognising and detecting the movement, acceleration and position (inclination on one or on the component of two orthogonal directions).

Said accelerometer (4), preferably of the piezoelectric type, produces one or more signals directly proportional to the acceleration undergone, or proportional to the movements and state of activity of the user, which are amplified by an integrated circuit and converted into digital values by the interface circuit (4.1), so that they can be read and interpreted by the control and command circuit (2).

Said control and command circuit (2) processes the signals received from the accelerometer (4) so as to average the envelope of the acceleration over a period which can be programmed from a few seconds to a few minutes, i.e. it interprets the state of activity of the user, and compares them with the patterns, parameters and tables stored in the memory (3).

Instead of one single accelerometer with three axes (4) it is possible to provide for the use of two or more accelerometers with two axes square to each other or three or more accelerometers with one single axis, arranged so as to be able to detect the acceleration in at least three directions not on the same plane.

Further indications can be obtained from the analysis of the signal frequency, reading for example the frequency of the user's step as he/she walks.

Further indications can be obtained by processing the signals relative to the three main axes, so as to determine the position, erect or lying down, and the side on which the user is lying.

According to the parameters set in the memory (3) and the signals received from the accelerometer (4), the control and command circuit (2) adopts specific values of one or more parameters of the neuromodulator, for example the amplitude of the stimulus delivered and its frequency.

The patterns, parameters and tables stored in the memory (3) can be read and set by the doctor via a telemetric connection between the neurostimulation device implanted and an external device at the disposal of the doctor. In this way the doctor can adapt operation of the new implantable neuromodulator to the needs and conditions of the user, also in subsequent phases.

The new analgesic neurostimulator provided with activation, de-activation and adaptation system according to the activity and position of the user offers considerable advantages.

The new neurostimulator does not necessarily require intervention of the user to be switched on or off.

The new neurostimulator autonomously recognises the state of wakefulness/activity and sleep of the user, adapting its intervention accordingly.

The new neurostimulator autonomously recognises the level of activity of the user, adapting the modes and intensity of intervention accordingly.

The new neurostimulator autonomously recognises the position of the user, erect or lying down, supine or prone or on one side, consequently modifying the modes and intensity of intervention according to the different arrangement of the nerve bundle in the dura mater with respect to the position of the neurostimulation electrode.

The new neurostimulator can provide for autonomous administration of the analgesic neural stimulation only in the periods required and with the necessary stimulation intensity. Consequently less energy is necessary compared to that required by a common analgesic neurostimulator.

The batteries used last longer than the batteries having the same capacity used in known analgesic neurostimulators.

Alternatively, it is possible to use lower capacity and therefore smaller batteries, consequently reducing the overall dimensions and weight of the entire neurostimulation device.

## Claims

1. Implantable analgesic neuromodulation device comprising a casing (1) housing at least one battery (6), one control and command circuit (2), and wherein said control and command circuit (2) is connected to one or more terminals (5), outside the casing (1), suitable for performing the neurostimulation, **characterised in that** it comprises a movement detection device or accelerometer (4) connected to said control and command circuit (2), and wherein said control and command circuit (2) analyses the signals generated by said movement detection device or accelerometer (4) and activates, interrupts or modifies administration of the neurostimulation.

2. Implantable analgesic neuromodulation device according to claim 1, **characterised in that** the movement detection device and the device for detecting the position of the user consist of at least one piezoelectric accelerometer (4) with at least two non-parallel axes.

3. Implantable analgesic neuromodulation device according to the preceding claims, **characterised in that** it comprises a memory (3) which stores patterns, parameters and neurostimulation application tables, such as switch-on, switch-off, modulation relative to two or more states of activity of the user, and wherein the electronic control and command circuit (2) compares the data read by the accelerometer (4) with said patterns, parameters and tables stored in the memory (5), consequently administering the optimal neural electrostimulation.

4. Implantable analgesic neuromodulation device according to claims 1, 2, **characterised in that** it comprises a memory (3) which stores patterns, parameters, neurostimulation application tables, such as switch-on, switch-off and modulation relative to two or more positions of the user, and wherein the electronic control and command circuit (2) compares the data read by the accelerometer (4) with said patterns, parameters and tables stored in memory (5), administering the scheduled neural electrostimulation.

5. Implantable analgesic neuromodulation device according to the preceding claims, **characterised in that** the electronic control and command circuit (2) processes the signals received from the accelerometer (4) so as to average the envelope of the acceleration over a period that can be programmed from a few seconds to a few minutes.

6. Implantable analgesic neuromodulation device according to the preceding claims, **characterised in that** the electronic control and command circuit (2) processes the signals received from the accelerometer performing a frequency analysis.
